# EUROPEAN PATENT SPECIFICATION

(11) **EP 1 882 944 B1**
(45) Date of publication and mention of the grant of the patent: **18.03.2009**
(21) Application number: 06118062.6
(22) Date of filing: 28.07.2006
(51) Int. Cl.: G01N 33/68

(54) **Method for the detection of amyloid-beta oligomers in body fluids**
Verfahren zum Nachweis der amyloid-beta Oligomere in Körperflüssigkeiten
Méthode de détection des oligomers d'amyloid-beta dans des fluides corporels

(43) Date of publication of application: 30.01.2008
(73) Proprietor: VISTA VENTURES GmbH, 80634 München (DE)
(72) Inventor: NAVARRETE SANTOS, Alexander, 06108 Halle (Saale) (DE); BÖHM, Gerald, 97072 Würzburg (DE)
(74) Representative: Behnisch, Werner

(56) References cited:
- WO-A-99/15903
- WO-A-99/57566
- WO-A2-20/05025516
- US-A1- 2005 069 935
- US-A1- 2006 166 275
- BACSKAI BRIAN J ET AL: "Fluorescence resonance energy transfer determinations using multiphoton fluorescence lifetime imaging microscopy to characterize amyloid-beta plaques." JOURNAL OF BIOMEDICAL OPTICS. JUL 2003, vol. 8, no. 3, July 2003 (2003-07), pages 368-375, XP002405820 ISSN: 1083-3668
- WALL J ET AL: "FLOW CYTOMETRIC CHARACTERIZATION OF AMYLOID FIBRILS" METHODS IN ENZYMOLOGY, ACADEMIC PRESS INC, SAN DIEGO, CA, US, vol. 309, 1999, pages 460-466, XP001006253 ISSN: 0076-6879
- GLABE C G: "Conformation-dependent antibodies target diseases of protein misfolding" TRENDS IN BIOCHEMICAL SCIENCES, ELSEVIER, HAYWARDS, GB, vol. 29, no. 10, October 2004 (2004-10), pages 542-547, XP004575216 ISSN: 0968-0004

## Description

### Field of the Invention:

The present invention relates to a method for the detection of a marker of the Alzheimer's disease, namely the amyloid β oligomers in human CSF and other body fluids.

### Background of the invention:

Alzheimer's disease (AD) is the most common neurodegenerative dementia with an average death prognosis of 7 years¹. Ongoing clinical studies point out promising possibilities for the treatment of this disease². For ideal therapy and timely conservation of essential cognitive functions, however, a diagnostic tool for the early detection of AD is a pre-requisite.

Recent work shows that oligomeric assemblies of Aβ are neurotoxic in cell culture and in vivo as they are able to inhibit long-term potentiation^{3,4}. Such low-molecular-weight Aβ oligomers were also shown to induce transient deficits in cognitive function⁵. To further demonstrate the adverse effect of oligomers on nerve cell function, immunotherapy was successfully used to neutralize Aβ oligomers, thereby restoring synaptic plasticity in vivo⁶. Further evidence pointing to Aβ oligomers as the neurotoxic species in AD is that these structures were also found in human brain where their concentration is up to 70-fold higher in AD patients compared to non-demented controls⁷⁻⁹.

It could thus be demonstrated that the severity of the disease correlates with oligomer concentration rather than with number of plaques¹⁰⁻¹² and the presence of globular Aβ oligomers in the brain is suggested to be an early pathological event in AD¹³. Hence, the search for Aβ oligomers was extended to human CSF and recent research work demonstrated the presence of low amounts of stable Aβ oligomers also in this body fluid¹⁴⁻¹⁷. As the concentration of oligomers was consistently higher in CSF of AD patients compared to non-demented age-matched controls, this points toward a correlation between the levels of oligomers and the state of the disease¹⁷, making βthem a possible biomarker and suitable target for the early detection of AD.

A sensitive method for the detection and accurate quantification of Aβ oligomers is thus required. One such method is the recently described bio-barcode assay for the measurement of amyloid-β-derived diffusible ligands (ADDLs) in CSF¹⁷. Although this method is quite sensitive, the procedure includes a relatively high number of critical steps that may affect the general performance of the assay.

D1: BACSKAI BRIAN J ET AL: "Fluorescence resonance energy transfer determinations using multiphoton fluorescence lifetime imaging microscopy to characterize amyloid-beta plaques." JOURNAL OF BIOMEDICAL OPTICS. JUL 2003, vol. 8, no. 3, July 2003 (2003-07), pages 368-375, XP002405820 ISSN: 1083-3668 describes the use of FRET-measurements in multiphoton fluorescence lifetime imaging microscopy to characterise amyloid-beta plaques.

D2: US 2005/069935 A1 describes a method for characterising associates and aggregates like amyloid-beta-oligomers by using fluorescence-activated cell sorter.

D3: WO 99/15903 A describes a method for measuring the association of substructures of pathological protein deposits like amyloid-beta peptide oligomers by using FCS-measurement methods.

### Problem of the Invention:

It is therefore a problem of the invention to provide a highly sensitive method for the detection and accurate quantification of Aβ oligomers comprising only a small and limited number of steps which can be easily controlled by the experimentator on the one side and, on the other side, provides highly reliable and reproducible results at reasonable costs in order to be used on a commercial basis.

### Summary of the Invention:

The invention provides a method for the detection of amyloid-β (Aβ) peptide oligomers in body fluids comprising the following steps:
a) providing a sample of a body fluid to be tested with respect to the presence of amyloid-β peptide oligomers;
b) demasking the epitopes responsible for antibody binding on said amyloid-β peptide oligomers by removing proteins attached to said amyloid-β peptide oligomers;
c) contacting said sample after said demasking step with one antibody comprising an antibody population binding to one epitope on said amyloid-β peptide oligomer, one part of the antibody population being labelled with a first fluorescence marker and the other part of the antibody population being labelled with a second fluorescence marker,
   or contacting said sample after said demasking step with at least two antibodies binding to at least two different epitopes on said amyloid-β peptide oligomers, the first antibody being labelled with a first fluorescence marker and the at least second antibody being labelled with a second fluorescence marker,
   wherein said first fluorescence marker acts as donor transferring its energy to said second fluorescence marker acting as acceptor;
d) determining the intensity of the fluorescence resonance energy transfer signal emitted by said fluorescence labelled sample to detect amyloid-β peptide oligomers present in said body sample.

Preferred embodiments and advantages will become apparent from the following detailed description including the experimental section, the drawings and the claims.

### Brief description of the Figures:

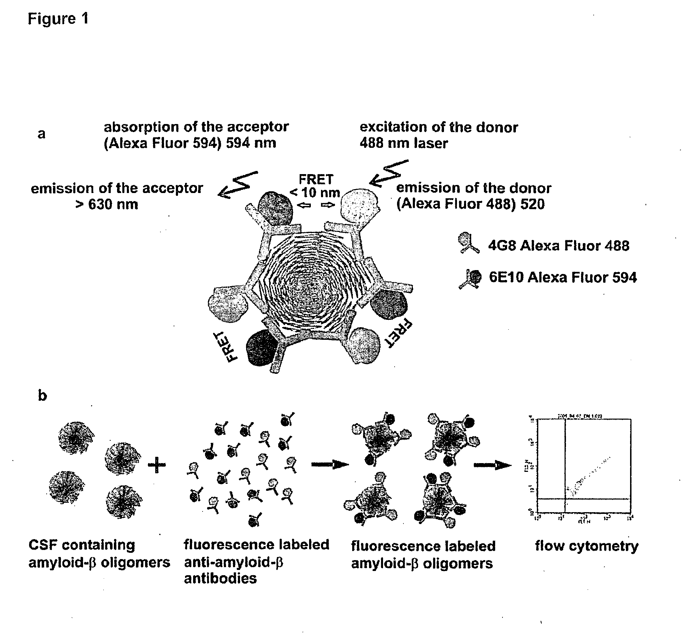

**Figure 1** Principle of the assay. (**a**) Amyloid-β oligomers are detected using two specific anti-amyloid β mAbs and FRET. The donor antibody is the clone 4G8 labeled with Alexa Fluor 488 and the acceptor antibody is the clone 6E10 Alexa Fluor 594 labeled. (b) The assay consists of one step: dilution of the CSF sample in detection buffer containing the labeled antibodies. After incubation in the dark, the amyloid-β oligomers are detected by flow cytometry.

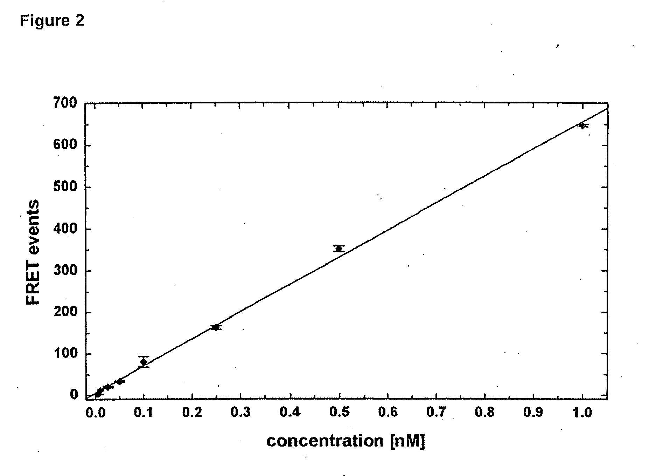

**Figure 2** Sensitivity of the assay. The sensitivity of the assay was determined by titration of *in vitro* assembled Aβ fibrils in the concentration range from 1 nM down to 0.0 nM. In this range the assay is linear. Because the concentration of the fibrils was estimated from the starting monomer concentration used for the assembling into fibrils and one fibril correspond to 100-1000 monomer, the real concentration of the measured fibril is to much lower doing the detection limit in the femtomol range.

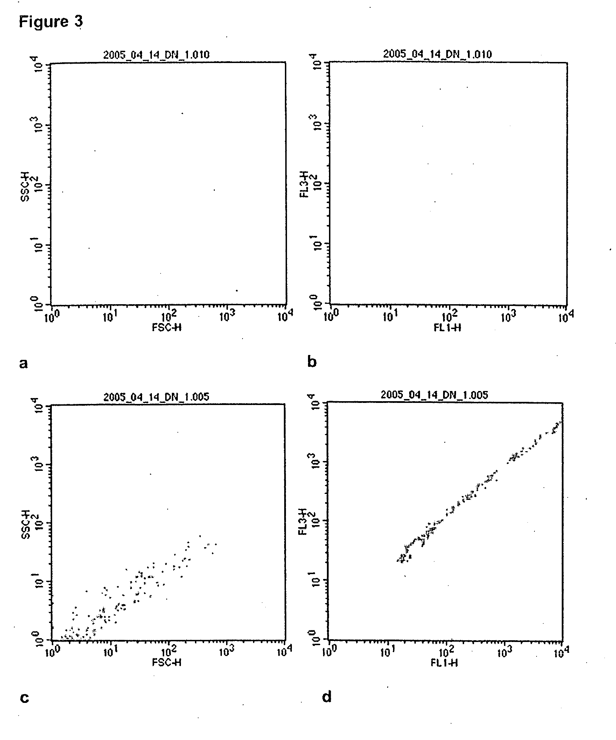

**Figure 3** Aβ monomers are not detected by flow cytometry. In order to check whether monomer of Aβ can be detected by flow cytometry, 1 nM of Aβ monomer 1-42 (Bachem) treated as described by Dahlgren et al. was incubated with the donor:acceptor antibody pair and analyzed by flow cytometry. No signals were detected in any case (FSC vs SC; FL1vs FL3 Dot plots; **a, b**). In contrast to Aβ monomer when 1 nM of *in vitro* assembled Aβ fibrils (seedless amyloid-(1-42) was dissolved in DMSO (Sigma-Aldrich) to 100 µM, diluted in PBS to 0.5 µM and incubated for 72 hours at 37 °C and the used concentration of the fibrils was based on the starting monomer concentration) FRET events were detected (FSC vs SCC; FL1 vs FL3; c,d) showing that with the assay it is possible to discriminate between monomeric Aβ (no detection possible) and Aβ oligomerized.

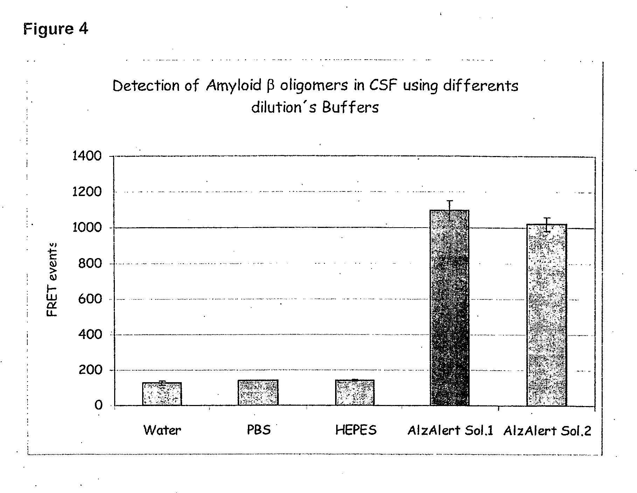

**Figure 4** Demasking of amyloid β oligomers in CSF. The use of the solution 1 and solution 2 efficiently demasks the amyloid oligomers allowing an effective detection by flow cytometry.

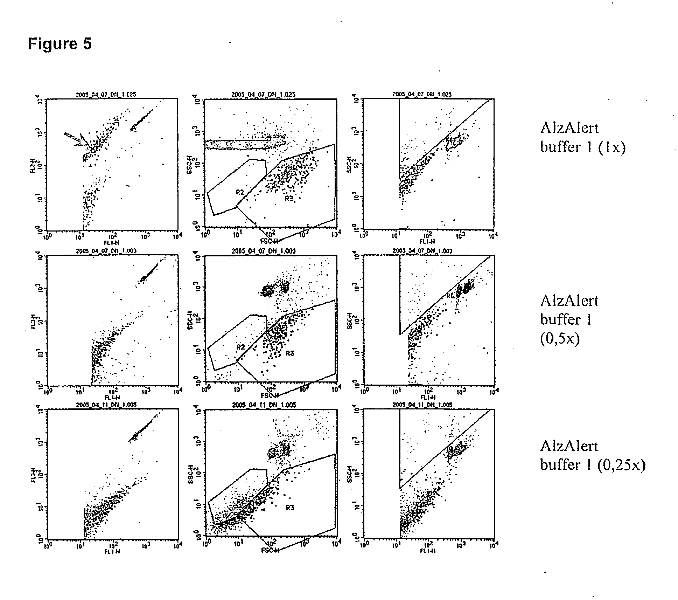

**Figure 5** Optimization of the detection's buffer. The solution 1 in a 0.5x concentration is optimal for the detection of the oligomers.

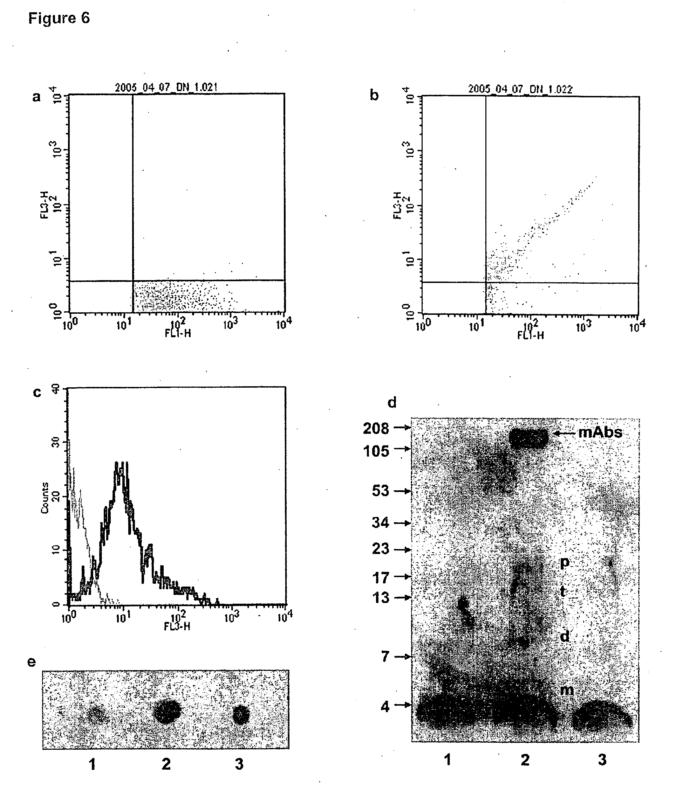

**Figure 6** Validation of the assay. (**a**) When CSF sample is incubated with the donor antibody (4G8 Alexa Fluor 488) only events in the channel of the FL1 are detected. (**b**) By incubation with the donor:acceptor pair of antibodies FRET events (FL1 and FL3) are detected. (**c**) A histogram analysis shows the shift of the events detected (events without FL3; green curve) into the FL3 channel (events with FL3; red curve). (d) When sorted CSF pool was analyzed by Western blot under non-reducing conditions, oligomers of Aβ were detected (lane 2). In 20 µl of CSF without sorting no oligomers were detected (lane 3). As control for the molecular mass of the bands detected 50 pg of amyloid-β monomer were loaded (Bachem, Switzerland; lane 1). (**e**) A Dot blot of the sorted CSF with the anti-oligomer specific antibody A11 further confirms the presence of oligomers in CSF (lane 3). As a control for the dot blot, 5 µl of brain homogenate from a healthy individual (lane 1) or from an AD patient (lane 2) were loaded.

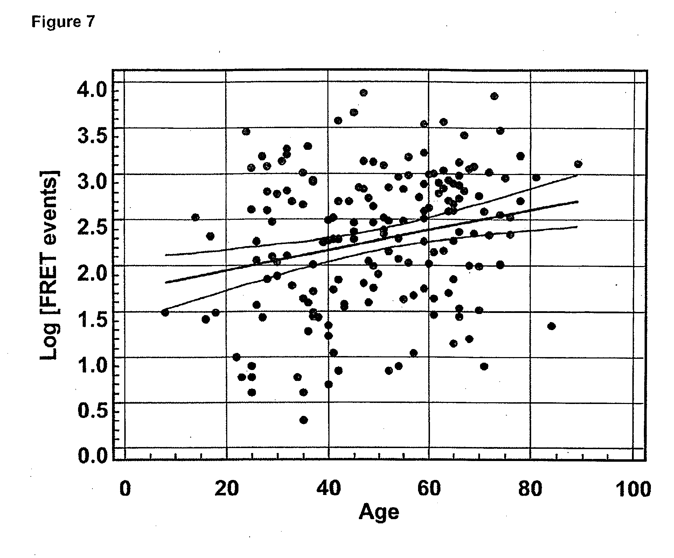

**Figure 7** Clinical performance of the assay. Analysis of 174 CSF samples from non-demented subjects aroused a positive correlation between the age and the amounts of Aβ oligomers detected. Scatter diagram of Aβ oligomers (FRET events) versus age. Analysis of 174 CSF samples from non-demented control subjects reveals a positive correlation (rho = 0.22; p = 0.0036) between age and concentration of Aβ oligomers. All values are mean values of two independent measurements. The linear regression and 95% confidence intervals are represented as solid lines.
**Figure 8** Stability of natural Aβ oligomers, reproducibility and clinical performance of the assay. **(a)** Effect of freeze/thaw cycles on the detection of natural Aβ oligomers in CSF. The concentration of the Aβ oligomers detected in a CSF pool was not altered by the application of three freeze/thaw cycles. **(b)** Analysis of seven different CSF samples by flow cytometry. All samples were analysed in duplicate and values are represented as mean values and standard deviations. **(c)** Western blot of the CSF samples analysed in **b.** There is no correlation between the amounts of Aβ as detected by Western blot and oligomer content.

### Detailed description of the Invention:

The invention is directed to a method for detecting amyloid-β peptide oligomers in body fluids. The expression "body fluids" covers all kinds of fluids which occur in the body of a vertebrate. Typical examples are blood, urine, tears, saliva, and cerebrospinal fluid, which is particularly preferred. All other kinds of body fluids may also be used in order to test them with respect to the presence of oligomeric amyloid-β peptides.

The body fluid is preferably taken from humans. However, all other kind of vertebrate animals may be tested in accordance with the present method including cattle, horses, dogs, cats and rodents like mice, rats and rabbits. Particularly, all humans showing first incidence of AD or other neurodegenerative diseases may be tested.

In a second step, it has been found to be crucial to remove any proteins being attached to the amyloid-β peptides oligomers from the peptides before contacting them with antibodies. The removal of proteins attached to the Aβ is called "demasking". In order to receive a reliable test result, at least any proteins attached with the epitopes of Aβ have to be eliminated. Preferably, all attached foreign proteins are removed.

The demasking step is preferably performed by contacting the body fluids with a detergent, preferably an anionic detergent or a combination of detergents. Typical examples for anionic detergents are SDS (sodium dodecyl sulfate), Triton, for instance Triton X-100 (t-Oct-C₆H₄-(OCH₂CH₂)xOH: t-octyl phenoxy polyethoxy ethanol), NP-40 (Nonidet P-40, ethyl phenyl polyethylene glycol), deoxycholates, particularly their sodium salts. It is also possible to use other detergents like cationic, non-ionic and amphoteric detergents. In a further embodiment, other demasking agents able to denature proteins are used. Examples are basic or acidic reagents. However, one disadvantage of using for instance basic reagents is, that they have to be removed before adding the monoclonal antibody. Generally all kind of detergents are applicable provided that they remove any proteins, particularly proteins attached with the epitopes, from Aβ, e.g, albumin, Apolipoprotein E. In a further embodiment, mixtures of detergents are used.

Typically, the body fluids are diluted in a buffer containing for instance Hepes, sodium chloride and/or Tris-HCl. The concentration of the buffer ingredients may be adjusted by the researcher. Typically, the detergent concentration is in the range of 0.1-1.0 percent by weight, preferably 0.2-0.7 percent by weight. Preferably protease inhibitors are added to the demasking solutions; in a still further embodiment also the antibody containing solution comprises protease inhibitors; this is particularly due in the most preferred embodiment wherein the demasking and the antibody incubation steps are performed in one step (batch). Examples for protease inhibitors are aprotinin, bestatin, EDTA, PMSF, leupeptin. Preferred concentrations of the ingredients in the buffer solutions the are described below. However, it has to be emphasized that the persons skilled in the art are able to adapt the kind of ingredients of the buffer as well as the concentration of the ingredients in accordance with the particulars of the test method.

### Preferred demasking solutions:

### Solution 1 is:

0.4-0.6, pref. 0.5 wt.-% NP-40
0.2-0.3, pref. 0.25 wt.-% sodium deoxycholate
0.01-0.5, pref. 0.05 wt.-% SDS
100-200, pref. 150 mM NaCl
20-100, pref. 50 mM Hepes

### Solution 2 is:

10-50, pref. 25 mM Tris-HCl pH8
0.4-0.6, pref. 0.5 wt.-% Triton X-100
0.4-0.6, pref. 0.5 wt.-% NP-40

Protease inhibitors are to be added in accordance with the experimental needs.

Examples for detergents and other demasking agents are anionic detergents, e.g SDS, Na-deoxycholate; nonionic detergents, e.g. Triton X-100, Tween 20, Nonidet P-40; zwitterionic detergents, e.g., CHAPS, and cationic detergents, e.g., tetradecyl trimethyl ammonium bromide (TTAB), and/or other denaturing demasking agents, preferably basic or acidic reagents, e.g. formic acid, guanidine hydrochloride or urea or alkali hydroxides.

The buffer solution containing the detergents and the body fluid is incubated for about 15 min to 3 h, preferably 30-90 min at a temperature of about 10-40°C, preferably 15-37°C, further preferably 17-30°C. The incubation time as well as the temperature may be adjusted by the skilled artisan in accordance with the particular test to be performed, for instance with respect to the body fluid to be tested, the detergent which is used etc.

As preferred concentration for the detergent about 0.01-2 percent by weight, particularly preferred 0.02-1 percent by weight is suggested. The concentration is lower than the critical micelle concentration (0.5 - 5%).

After said demasking step, the sample is contacted with one or more antibodies binding to the epitopes characteristic for the Aβ peptide. In a preferred embodiment, the demasking step (b) and the contacting step (c) are performed in one step (in one batch).

Typical antibodies to be used are monoclonal antibodies which recognize epitopes within the amino terminus of the Aβ peptide, preferably within amino acid (aa) positions 1-24. Particularly preferred epitopes comprise amino acids (aa) 4-13 or 17-24. However, also other epitopes characteristic for the Aβ peptide may be used extending to regions of the Aβ peptide other than the amino terminus. Examples are the epitope at aa 1-5; epitope at aa 1-11; epitope at aa 1-7; epitope at aa 15-24; epitope at aa 3-9; epitope at aa 11-26; epitope at aa 4-10 ; epitope at aa 13-28 ; epitope at aa 1-10; epitope at aa 31-40; epitope at aa 8-17; epitope at aa 15-30; epitope at aa 17-24; epitope at aa 1-28; epitope at 12-28; epitope at aa 17-42; epitope at aa 20-40: epitope at aa 37-42epitope at aa 8-17 ; epitope at aa 11-28; epitope at aa 1-6; epitope at aa 8-17; epitope at aa 12-28; epitope at aa 25-35; epitope at aa 15-30; epitope at aa 17-26; epitope at aa 1-12; epitope at aa 32-40; epitope at aa 33-42.

While particularly preferred two antibodies are used, preferably covering amino acid positions 4-13 and 17-24, in further embodiments of the invention also more than two, for instance three or four antibodies or only one antibody may be used.

If one antibody is used, one part of the antibody population is labelled with a first fluorescence marker while the other part of the antibody population is labelled with a second fluorescence marker. The markers act as donor and acceptor in order to fulfil the criteria of the FRET technology. As one Aβ oligomer provides the same epitope several times, the FRET technology can be used. The term "one antibody" is to be understood to cover a homogenous, i.e. identical population of monoclonal antibodies.

In a still further embodiment, two or more than two antibodies are used, and each antibody is labelled with a different fluorescence marker or with two fluorescence markers only.

The antibodies used are specifically labelled with fluorescent markers. Fluorescently labelled antibodies are well-known in the art and may be purchased commercially. Fluorescent staining of proteins is well-known in the art, and means to detect fluorescently labelled antibodies are known. The fluorescent dyes typically bind by non-covalent or covalent interactions with the protein. In accordance with the invention any fluorescent dye may be used which is able to bind to the monoclonal antibodies in the present invention and which can be detected by cytometric or photometrical methods, for instance by flow cytometry; particularly preferred are fluorescent dyes which can be used in FRET.

In a particularly preferred embodiment, the method of the present invention utilizes flow cytometry combined with fluorescence resonance energy transfer (FRET). FRET is an excellent tool for determining distances and supramolecular organization of biomolecules (Figure 1a). FRET is a special phenomenon in fluorescence spectroscopy during which energy is transferred from an excited donor molecule to an acceptor molecule under favorable spectral and spatial conditions¹⁹. For detecting Aβ, one epitope is labelled with a donor and the other epitope with an acceptor. The most popular FRET pair for practical use is CFP and YFP. Both are color variants of green fluorescent protein GFP. When the donor and the acceptor are quite distant from each other, the donor emission is detected on the donor excitation while, on the other hand, when the donor and acceptor are in close proximity due to the interaction of a donor and the acceptor, the acceptor emission is predominately observed because of the intermolecular FRET from the donor to the acceptor. For the combined FRET effect, the emission peak of the donor must overlap the excitation peak of the acceptor. In FRET, light energy is added at the excitation frequency for the donor fluorophor, which transfers some of its energy to the acceptor, which then re-emits the light at its own emission wavelengths. The net result is that the donor emits less energy than it normally brought (since some of the energy gets transferred to the acceptor instead), while the acceptor emits more light energy at its excitation frequency (because it is getting extra energy input from the donor fluorophor).

The benefit of FRET technology is its excellent resolution. FRET only occurs when the two fluorophors are within 2-10 nm of each other meaning that the fluorophors must be brought together via a very close spatial distance. If the fluorophors are more than 20 nm apart, no signal will be observed. Therefore, it is important to select epitopes in A-β if the FRET effect is utilized in combination with flow cytometry.

In a typical embodiment of the invention, two monoclonal anti-Aβ antibodies that recognize different epitopes of the Aβ peptide sequence are labeled with the fluorescence dyes Alexa Fluor 488 (mAb 4G8; raised against Aβ 17-24) and Alexa Fluor 594 (mAb 6E10; raised against Aβ 4-13). Other fluorescence dyes such as ATTOs or Cy can be suitable. Generally, all known fluorescence dyes can be used if they are able to provide the FRET effect as donor/acceptor molecules. For instance the mAb 4G8-Alexa Fluor 488 corresponds to the donor molecule and the mAb 6E10-Alexa Fluor 594 functions as the acceptor molecule (**Figure 1a**). With this donor/acceptor combination, Aβ monomers are not detectable in the present, which is due to the low amount of fluorophores that are able to bind to Aβ monomers (a maximum of two antibodies per monomer) and the resulting very low intensity of the emitted fluorescence and FRET signal (Figure 3). In contrast, oligomeric structures of Aβ are able to bind sufficient amounts of antibody molecules and give thus rise to a fluorescence signal strong enough to be detected by flow cytometry (**Figure 3**). The information content of the fluorescence signal is increased by FRET, as it allows a differentiation of unspecific binding of the mAb 4G8 to other molecules. Hence, signals from the 6E10-Alexa Fluor 594 antibody are only observed if the distance between both antibodies is closer than 10 nm, the Förster distance that allows energy transfer between donor and acceptor fluorophores.

In the last step, the fluorescence resonance energy transfers signal emitted by the fluorescence labelled sample is then detected by well-known means. Examples are flow cytometry or photometrical methods. All kind of methods and means for detecting the fluorescence signal are applicable.

Preferred embodiments of the invention are now described with respect to the examples. It is to be noted that the application is not limited to those embodiments described below.

### METHODS

**Collection of cerebrospinal fluid.** CSF was obtained from 174 neurological patients (mean age 49.3 years; range 8-89) with diagnoses such as multiple sclerosis. All samples were obtained by lumbar puncture, frozen within 2 h and stored at -85°C before analysis; repeated freeze/thaw cycles were avoided.

**Fluorescence labeling of antibodies.** The anti-Aβ antibodies 4G8 and 6E10 (Chemicon International) were labeled with the fluorescence dyes Alexa Fluor 488 or Alexa Fluor 594 according to the manufacture's instructions (applying the respective Monoclonal Antibody Labeling Kits; Invitrogen).

**Sample preparation.** 200 µl of CSF were diluted with 200 µl of 1 x solution 1 (5 x solution 1 contains 250 mM HEPES; 750 mM NaCl, 0.25% SDS, 1.25% Nadeoxycholate, 2.5% NP-40 alternative (Calbiochem) and 1 tablet of protease inhibitor cocktail Complete^{™} Mini (Roche Applied Science) per 2 ml of 5 x Solution 1). Then the fluorescence labeled antibodies were added to concentrations of 2 nM and 8 nM for donor antibody (4G8, labeled with Alexa-Fluor 488) and acceptor antibody (6E10, labeled with Alexa-Fluor 594), respectively. After incubation (90 minutes at room temperature, protected from light), samples were analyzed on a FACS Calibur (BD Biosciences) as described below.

**Flow cytometry.** For the detection of Aβ-oligomers a FACS Calibur flow cytometer equipped with a 15mW 488nm air-cooled argon-ion laser (BD Biosciences) was used. The oligomer particles were gated in logarithmic forward/sideward scatter Dot plots (FSC vs SSC). The green or red fluorescence of the dyes Alexa Fluor 488 and Alexa Fluor 594 was detected by the corresponding FL1 and FL3 (logarithmic scale) photomultipliers through 530/30 or 670LP bandpass filters, respectively. To avoid differences in the measurement due to variation in the CSF samples, all samples were measured in TruCount Tubes (BD Biosciences) and analysis was stopped after 28,000 beads were counted.

**Sorting.** Sorting of the oligomer-specific region was performed on a FACS Vantage cell sorter (BD Biosciences), applying a threshold to the FL1 channel. The population of interest was gated in a Dot plot of FL1 vs FL3 and only events with a FRET signal were sorted.

**Western blot.** 20 µl of a CSF sample or 25 µl sorted CSF were applied to 16% Tricine gels (Invitrogen), run for 2 hours, and transferred onto t PVDF membranes. Membranes were boiled for 5 min in PBS and blocked for 2 hours in 5% skimmed milk powder in TBS-T (10mM Tris-HCl, pH 7.6 containing 150 mM NaCl and 0.1% Tween 20). Then the monochlonal anti-Aβ antibody 6E10 was applied and incubated over night. After washing the membranes in TBS-T, the bound antibody was visualized using HRP-conjugated secondary anti-mouse antibody and ECL detection (SuperSignal West Femto Substrate, Pierce).

**Dot blot**. For dot blot analysis the samples were directly applied to the nitrocellulose membrane and air-dried. The membrane was then processed to determine the presence of Aβ oligomers using the anti-oligomer specific antibody A-11 (Biosource) according to the manufacturer's protocol.

**Isolation of amyloid-β oligomers from AD brain homogenate.** Frontotemporal brain tissue from control persons and AD patients was kindly provided by the German brain bank. Oligomers from control and AD brains were isolated according to *Wiltfang et al*¹⁹.

**Statistical analysis.** Statistical analysis was performed using the MedCalc software and the Spearman's rank correlation coefficient.

**Preparation of seedless Aβ (1-42).** Aβ (1-42) was purchased from Bachem, seedless treated as described by Dahlgren et al. (Dahlgren, K.N. et al. Oligomeric and fibrillar species of amyloid-beta peptides differentially affect neuronal viability. J. Biol. Chem. 277, 32046-32035 (2002)), further purified by RP-HPLC (column: Source 5RPC 4.6/150 ST Amersham); solvent A: 0.1 % NH₄OH (25%) in H₂O pH9.0; solvent B: 60% acetonitril, 40% solvent A; gradient: 25-56% solvent B in 31 min; flow rate: 1.0ml/min) and lyophilized.

**Generation of in vitro fibrilis.** Seedless Aβ (1-42) was dissolved in DMSO (Sigma-Aldrich) to 1 mM, diluted to 100 µm into 10 mM HCl and incubated for 24 hours at 37°C (Dahlgren et al 2002). For storage, fibril preparations were diluted to 5 µM into 100 mM Hepes, 2.5 mM DTT, 5 mM EDTA, 250 mM NaCl, 2% glycerine, pH 7.6 and frozen at - 80°C.

### Examples:

**Example 1:** The sensitivity of the assay was determined by titration of *in vitro* assembled Aβ fibrils in the concentration range from 1 nM down to 0.0025 nM. In this range the assay is linear. Because the concentration of the fibrils was estimated from the starting monomer concentration used for the assembling into fibrils and one fibril correspond to 100-1000 monomer the real concentration of the measured fibril is much lower, doing the detection limit in the femtomol range (**Figure 2**).

**Example 2:** In order to check whether monomer of Aβ can be detected by flow cytometry 1 nM of Aβ monomer 1-42 (Bachem) treated as described by Dahlgren et al.¹⁵ was incubated with the donor:acceptor antibody pair and analyzed by flow cytometry. No signals were detected in any case (FSC vs SC; FL1vs FL3 Dot plots; **Figure 3a****,b**). In contrast to Aβ monomer when 1 nM of *in vitro* assembled Aβ fibrils (seedless amyloid-(1-42) was dissolved in DMSO (Sigma-Aldrich) to 100 µM, diluted in PBS to 0.5 µM and incubated for 72 hours at 37°C and the used concentration of the fibrils was based on the starting monomer concentration) FRET events were detected (FSC vs SCC; FL1 vs FL3) showing that with the assay there is possible to discriminate between monomeric Aβ (no detection possible) and Aβ oligomerized (**Figure 3c****,d**).

**Example 3:** For flow cytometry measurements, CSF was diluted into different buffer solutions. Only dilution of CSF into a buffer containing specific amounts of several detergents allows an optimal detection of amyloid β oligomers (solution 1 and solution 2). The pre-treatment procedure is therefore an essential and highly critical step for the measurement of amyloid β oligomers. As shown in Figure 4, the use of water or common buffers (PBS, HEPES) for dilution of CSF only allow the detection of a minute fraction of oligomers present in the sample. The reason for this inefficient detection of oligomers is a so-called epitope-masking that competes with antibody binding.

**Example 4:** As evident from example 3, solution 1 is superior for the detection of amyloid β oligomers in CSF. This buffer solution was therefore selected for further measurements and its concentration was optimized. As shown in Figure 5 (top), when used as a 1-fold concentrated solution, solution.1 increases the background of the measurements (arrow). A similar result is obtained when the buffer concentration is reduced to 0.25-fold - then most of the detected signal is unspecific background (**Figure 5**, bottom). A 0.5-fold concentrated solution.1, however, satisfactorily de-masked the oligomers without increasing the unspecific background signal (**Figure 5**, middle). This example thus demonstrates that the detection of oligomers in CSF essentially depends on sample pre-treatment (i.e. selection of an appropriate detergent mixture) and hence buffer conditions.

**Example 5:** In the current setup, 200 µl of CSF are diluted with 200 µl of solution 1, followed by incubation with the FRET antibody mixture. Although we used 200 µl of each CSF sample, this volume is not limiting and can be increased if a higher sensitivity is required. To better visualize the FRET effect, we first incubated human CSF with the antibody 4G8-Alexa Fluor 488 alone. In the subsequent flow cytometric analysis, an oligomer-specific population was detected showing events in the fluorescence 1 channel only (**Figure 6a**; FL1-H). Addition of the FRET acceptor antibody 6E10-Alexa Fluor 594 then induced an additional fluorescence signal in the fluorescence 3 channel (**Figure 6b****-c**; FL3-H). This gain of an Alexa Fluor 594 specific emission demonstrates the binding of both antibodies in close proximity to each other (< 10nm), allowing energy transfer from the Alexa Fluor 488 donor to the Alexa Fluor 594 acceptor fluorophore. Although the sensitivity for the detected signal increases upon addition of the acceptor antibody, the overall number of specific events simultaneously decreases, possibly due to a sterical competition of both antibodies for their respective epitopes as well as a FRET induced fluorescence quenching of the acceptor fluorophores. Since the probability for non-specific binding of both antibodies within a distance closer than 10 nm is extremely low, these data suggest that the detected events are indeed Aβ oligomers. To further validate this assumption, a pool of CSF (6 ml) was prepared and analyzed as described before. The events detected in the region of interest, however, were sorted with a FACS Vantage cell sorter and subsequently analyzed by Western blot and Dot blot. As shown in Figure 6d, Aβ assemblies ranging from monomers to pentamers could be detected by Western blot, confirming the sorted particles to be composed of the Aβ peptide (**Figure 6d**, lane 2). Comparison of the sample derived from sorting with a CSF sample directly loaded onto the gel shows that the higher molecular weight forms can only be detected in the sample enriched by sorting (Figure 6d, lanes 2-3). Since the assay does not detect Aβ monomers (Figure 3), this indicates the monomer band of the sorted material to originate from oligomeric structures. It can therefore be suggested that most of the oligomers found in CSF are not covalently crosslinked or SDS-resistant. Further proof for the detected Aβ oligomers was achieved by Dot blot analysis of the sorted population, resulting in a positive signal when probed with the oligomer-specific antibody A-11 (Figure 6e).

**Example 6:** In order to test the clinical assay performance, we finally extended our CSF analysis on samples obtained from 174 non-demented individuals with various neurological disorders (**Figure 7**). Evaluation of the data shows a large variation in the concentration of the detected oligomers. We found, however, a correlation between the age of the individuals and amounts of Aβ oligomers (rho = 0.22; p = 0.0036), showing for the first time that the Aβ oligomer concentration increases with age. This result supports the assumption that the equilibrium of soluble-to-insoluble Aβ is disturbed with age and provides an explanation for the observed age-dependent decrease of monomeric Aβ in CSF, namely the formation of oligomers.

**Example 7:** It is known that repeated freeze/thaw cycles of CSF lead to a decrease in the concentration of Aβ monomers²⁰. The absence of a reliable detection method, however, did render such studies impossible for oligomers. We therefore examined the effect of freeze/thaw cycles using a pool of CSF that was frozen at -80°C and thawed consecutively three times. The first value was measured before freezing and was set as 100%. In contrast to the situation observed for Aβ monomers²⁰, no significant effect was observed for the overall amount of oligomers for all applied freeze/thaw cycles (**Figure 8**).

We further investigated the reproducibility of our assay, exemplified for seven different CSF samples in Figure 8b. The standard deviation for these samples is 3.5 ± 2.1% (**Figure 8b**) and below 5% for the overall assay. As can also be seen from this Figure, two of the CSF samples were negative for Aβ oligomers, even through all samples contained approximately equal amounts of monomers as detected by Western blot. (**Figure 8c**). This demonstrates again that there is no correlation between oligomer content and concentration of Aβ as detected by Western blot or ELISA²¹ and underlines the advantage of the assay over other methods, i.e. specificity for oligomers, scalable sample volume and thus enhanced sensitivity.

As flow cytometry is a common method in routine diagnostics, the use of our assay could be a cheap alternative to other methods, particularly as it allows a high sample throughput and automation.

### References

1. Fitzpatricka, A.L., Kullerb, L.H., Lopezc, O.L., Kawasd, C.H. & Jaguste W. Survival following dementia onset: Alzheimer's disease and vascular dementia. J. Neurol. Sci. 229-230, 43-49 (2005).
2. Aisen, P.S. The development of anti-amyloid therapy for Alzheimer's disease: from secretase modulators to polymerisation inhibitors. CNS Drugs. 19, 989-996 (2005).
3. Walsh, D.M. et al. Naturally secreted oligomers of amyloid beta protein potently inhibit hippocampal long-term potentiation in vivo. Nature 416, 535-539 (2002).
4. Lambert, M.P. et al. Diffusible, nonfibrillar ligands derived from Abeta1-42 are potent central nervous system neurotoxins. Proc. Natl. Acad. Sci. USA. 95, 6448-6453 (1998).
5. Cleary, J.P. et al. Natural oligomers of the amyloid-beta protein specifically disrupt cognitive function. Nat. Neurosci. 1, 79-84 (2005).
6. Klyubin, I. et al. Amyloid β protein immunotherapy neutralizes Aβ oligomers that disrupt synaptic plasticity in vivo. Nat. Med. 6, 556-561 (2005).
7. Kuo, Y.M. et al. Water-soluble Abeta (N-40, N-42) oligomers in normal and Alzheimer disease brains. J. Biol. Chem. 271, 4077-4081 (1996).
8. Roher, A.E. et al. Morphology and toxicity of Abeta-(1-42) dimer derived from neuritic and vascular amyloid deposits of Alzheimer's disease. J. Biol. Chem. 271 20631-20635 (1996).
9. Gong, Y. et al. Alzheimer's disease-affected brain: presence of oligomeric A beta ligands (ADDLs) suggests a molecular basis for reversible memory loss. Proc. Natl. Acad. Sci. USA. 100, 10417-10422 (2003).
10. Lue, L.F. et al. Soluble amyloid beta peptide concentration as a predictor of synaptic change in Alzheimer's disease. Am. J. Pathol. 155, 853-862 (1999).
11. McLean, C.A. et al. Soluble pool of Abeta amyloid as a determinant of severity of neurodegeneration in Alzheimer's disease. Ann. Neurol. 46, 860-866 (1999).
12. Enya, M. et al. Appearance of sodium dodecyl sulfate-stable amyloid beta-protein (Abeta) dimer in the cortex during aging. Am. J. Pathol. 154, 271-279 (1999).
13. Barghorn, S. et al. Globular amyloid beta-peptide oligomer - a homogenous and stable neuropathological protein in Alzheimer's disease. J. Neurochem. 31, 834-847 (2005).
14. Vigo-Pelfrey, C., Lee, D., Keim, P., Lieberburg, I. & Schenk, D.B. Characterization of beta-amyloid peptide from human cerebrospinal fluid. J. Neurochem. 611, 1965-1968 (1993).
15. Pitschke, M., Prior, R., Haupt, M. & Riesner, D. Detection of single amyloid beta-protein aggregates in the cerebrospinal fluid of Alzheimer's patients by Fluorescence correlation spectroscopy. Nat. Med. 4, 832-834 (1998).
16. Walsh, D.M., Tseng, B.P., Rydel, R.E., Podlisny, M.B. & Selkoe, D.J. The oligomerization of amyloid beta-protein begins intracellularly in cells derived from human brain. Biochemistry 39, 10831-10839 (2000).
17. Georganopoulou, D.G. et al. Nanoparticle-based detection in cerebral spinal fluid of a soluble pathogenic biomarker for Alzheimer's disease. Proc. Natl. Acad. Sci. USA. 102, 2273-2276 (2005).
18. Dahlgren, K.N. et al. Oligomeric and fibrillar species of amyloid-beta peptides differentially affect neuronal viability. J. Bio.l Chem. 277, 32046-32053 (2002)
19. Wiltfang, J. et al. Elevation of beta-amyloid peptide 2-42 in sporadic and familial Alzheimer's desease and ist generation in PS1 knockout cells. J. Biol. Chem. 276, 42645-42657 (2001).
20. Schoonenboom N. S. et al. Effects of processing and storage conditions on amyloid beta (1-42) and tau concentrations in cerebrospinal fluid: implications for use in clinical practise. Clin. Chem. 51, 189-195 (2005).
21. Stenh, C. et al. Amyloid-beta oligomers are inefficiently measured by enzymelinked immunosorbent assay. Ann. Neurol. 58, 147-150 (2005).

## Claims

1. A method for the detection of amyloid-β peptide oligomers in body fluids comprising the following steps:
a) providing a sample of a body fluid to be tested with respect to the presence of amyloid-β peptide oligomers;
b) demasking the epitopes responsible for antibody binding on said amyloid-β peptide oligomers by removing proteins attached to said amyloid-β peptide oligomers;
c) contacting said sample after said demasking step with one antibody comprising an antibody population binding to one epitope on said amyloid-β peptide oligomer, one part of the antibody population being labelled with a first fluorescence marker and the other part of the antibody population being labelled with a second fluorescence marker,
or contacting said sample after said demasking step with at least two antibodies binding to at least two different epitopes on said amyloid-β peptide oligomers, the first antibody being labelled with a first fluorescence marker and the at least second antibody being labelled with a second fluorescence marker,
wherein said first fluorescence marker acts as donor transferring its energy to said second fluorescence marker acting as acceptor;
d) determining the intensity of the fluorescence resonance energy transfer signal emitted by said fluorescence labelled sample to detect amyloid-β peptide oligomers present in said body sample.

2. The method in accordance with claim 1, wherein said body fluid is a vertebrate body fluid, preferably from humans, cattle, horses, dogs, cats and rodents.

3. The method of claim 1 or 2, wherein said epitopes are located preferably within amino acid positions aa 1-24 within the amino terminus, and wherein the epitopes are preferably selected from the group consisting of epitope at aa 4-13, epitope at aa 17-24; epitope at aa 1-5; epitope at aa 1-11; epitope at aa 1-7; epitope at aa 15-24; epitope at aa 3-9; epitope at aa 11-26; epitope at aa 4-10 ; epitope at aa 13-28 ; epitope at aa 1-10; epitope at aa 31-40; epitope at aa 8-17; epitope at aa 15-30; epitope at aa 17-24; epitope at aa 1-28; epitope at 12-28; epitope at aa 17-42; epitope at aa 20-40: epitope at aa 37-42epitope at aa 8-17 ; epitope at aa 11-28; epitope at aa 1-6; epitope at aa 8-17; epitope at aa 12-28; epitope at aa 25-35; epitope at aa 15-30; epitope at aa 17-26; epitope at aa 1-12; epitope at aa 32-40; epitope at aa 33-42.

4. The method according to one or more of the preceding claims, wherein said demasking is performed by adding one or more detergents, preferably anionic detergents, e.g SDS, Na-deoxycholate; nonionic detergents, e.g. Triton X-100, Tween 20, Nonidet P-40; zwitterionic detergents, e.g., CHAPS, and cationic detergents, e.g., tetradecyl trimethyl ammonium bromide (TTAB), and/or other denaturing demasking agents, preferably basic or acidic reagents, e.g. formic acid, guanidine hydrochloride or urea or alkali hydroxides.

5. The method according to claim 4, wherein said detergent is used in a concentration lower than the critical micelle concentration, preferably at about 0.01 - 2%, preferably 0.02 - 1 % by weight.

6. The method according to one or more of the preceding claims, wherein the temperature of said demasking step b) and/or of said contacting step c) is in the range of about 15°C - 40°C, preferably 17°C - 30°C and wherein preferably the incubation with said demasking agents and/or said antibodies is for about 30 - 90 min.

7. The method according to one or more of the preceding claims, wherein said body fluid is selected from the group consisting of cerebrospinal fluid, blood, urine, tears and saliva.

8. The method according to one or more of the preceding claims, wherein said intensity of said fluorescence resonance energy transfer signal is determined by flow cytometry or photometrical methods.

9. The method according to claim 1, wherein steps (b) and (c) are performed simultaneously in one batch.

10. The method according to one or more of the preceding claims, wherein into the demasking solution and/or into the antibody containing solution protease inhibitors are added.

## Patentansprüche

1. Verfahren zum Nachweis von Amyloid-β-Peptidoligomeren in Körperflüssigkeiten, umfassend die nachfolgenden Schritte:
a) Bereitstellen einer Probe einer Körperflüssigkeit, die in Bezug auf das Vorliegen von Amyloid-β-Peptidoligomeren zu untersuchen ist;
b) Demaskieren der Epitope, die für die Antikörperbindung auf den Amyloid-β-Peptidoligomeren verantwortlicht sind durch Entfernen der Proteine, die an die Amyloid-β-Peptidoligomere angelagert sind;
c) In Kontaktbringen der Probe nach dem Demaskierungsschritt mit einem Antikörper, umfassend eine Antikörperpopulation, die an ein Epitop auf dem Amyloid-β-Peptidoligomer bindet, wobei ein Teil der Antikörperpopulation mit einem ersten Fluoreszenzmarker und der andere Teil der Antikörperpopulation mit einem zweiten Fluoreszenzmarker markiert sind,
oder in Kontaktbringen der Probe nach dem Demaskierungsschritt mit zumindest zwei Antikörpern, die an zumindest zwei unterschiedliche Epitope auf den Amyloid-β-Peptidoligomeren binden, wobei der erste Antikörper mit einem ersten Fluoreszenzmarker und der zumindest zweite Antikörper mit einem zweiten Fluoreszenzmarker markiert sind,
wobei der erste Fluoreszenzmarker als Donor wirkt, der seine Energie auf den zweiten Fluoreszenzmarker überträgt, der als Akzeptor wirkt;
d) Bestimmen der Intensität des Fluoreszenzresonanzenergietransfersignal, welches von der fluoreszenzmarkierten Probe emittiert wird, um die in der Körperprobe vorhandenen Amyloid-β-Peptidoligomere nachzuweisen.

2. Verfahren nach Anspruch 1, wobei die Körperflüssigkeit eine Vertebratenkörperflüssigkeit ist, bevorzugt aus Menschen, Vieh, Pferden, Hunden, Katzen und Nagern.

3. Verfahren nach Anspruch 1 oder 2, wobei die Epitope bevorzugt in den Aminosäurepositionen aa 1-24 im Aminoterminus lokalisiert sind, und wobei die Epitope bevorzugt ausgewählt werden aus der Gruppe, bestehend aus dem Epitop bei aa 4-13; Epitop bei aa 17-24; Epitop bei aa 1-5; Epitop bei aa 1-11; Epitop bei aa 1-7; Epitop bei aa 15-24; Epitop bei aa 3-9; Epitop bei aa 11-26; Epitop bei aa bei 4-10; Epitop bei aa 13-28; Epitop bei aa 1-10; Epitop bei aa 31-40; Epitop bei aa 8-17; Epitop bei aa 15-30; Epitop bei aa 17-24; Epitop bei aa 1-28; Epitop bei aa 12-28; Epitop bei aa 17-42; Epitop bei aa 20-40; Epitop bei aa 37-42; Epitop bei aa 8-17; Epitop bei aa 11-28; Epitop bei aa 1-6; Epitop bei aa 8-17; Epitop bei aa 12-28; Epitop bei aa 25-35; Epitop bei aa 15-30; Epitop bei aa 17-26; Epitop bei aa 1-12; Epitop bei aa 32-40; Epitop bei aa 33-42.

4. Verfahren nach einem oder mehreren der vorhergehenden Ansprüche, wobei die Demaskierung durch Zugabe ein oder mehrerer Detergentien, bevorzugt anionische Detergentien, beispielsweise SDS, Na-Deoxycholat; nicht-ionische Detergentien, z.B. Triton X-100, Tween 20, Nonidet P-40; zwitterionischer Detergentien, z.B. CHAPS, und kationischer Detergentien, z.B. Tetradecyltrimethylammoniumbromid (TTAB), und/oder anderer denaturierender Demaskierungsmittel, bevorzugt basischer oder saurer Reagentien, z.B. Ameisensäure, Guanidinhydrochlorid oder Harnstoff oder Alkalihydroxide, durchgeführt wird.

5. Verfahren nach Anspruch 4, wobei das Detergenz in einer Konzentration unterhalb der kritischen Micellenkonzentration, bevorzugt bei etwa 0,01 - 2%, bevorzugt 0,02 - 1 Gew.-%, eingesetzt wird.

6. Verfahren nach einem oder mehreren der vorhergehenden Ansprüche, wobei die Temperatur des Demaskierungsschritts b) und/oder des Kontaktierungsschritts c) im Bereich von etwa 15°C - 40°C, bevorzugt 17°C - 30°C liegt, und wobei bevorzugt die Inkubation mit den Demaskierungsmitteln und/oder den Antikörpern etwa 30 - 90 Min. beträgt.

7. Verfahren nach einem oder mehreren der vorhergehenden Ansprüche, wobei die Körperflüssigkeit ausgewählt wird aus der Gruppe, bestehend aus Cerebrospinalflüssigkeit, Blut, Urin, Tränen und Speichel.

8. Verfahren nach einem oder mehreren der vorhergehenden Ansprüche, wobei die Intensität des Fluoreszenzresonanzenergietransfersignals mittels Durchflusscytometrie oder photometrischer Verfahren bestimmt wird.

9. Verfahren nach Anspruch 1, wobei die Schritte (b) und (c) gleichzeitig in einem Ansatz durchgeführt werden.

10. Verfahren nach einem oder mehreren der vorhergehenden Ansprüche, wobei in die Demaskierungslösung und/oder in die Antikörper enthaltende Lösung Proteaseinhibitoren zugegeben werden.

## Revendications

1. Procédé pour la détection d'oligomères de peptide amyloïde-β dans des fluides corporels comprenant les étapes suivantes:
a) fournir un échantillon d'un fluide corporel à évaluer en ce qui concerne la présence d'oligomères de peptide amyloïde-β;
b) démasquer les épitopes responsables de la liaison d'anticorps sur lesdits oligomères de peptide amyloïde-β en enlevant les protéines attachées auxdits oligomères de peptide amyloïde-β;
c) mettre en contact ledit échantillon après ladite étape de démasquage avec un anticorps comprenant une population d'anticorps se liant à un épitope sur ledit oligomère de peptide amyloïde-β, une partie de la population d'anticorps étant étiquetée avec un premier marqueur de fluorescence et l'autre partie de la population d'anticorps étant étiquetée avec un second marqueur de fluorescence,
ou mettre en contact ledit échantillon après ladite étape de démasquage avec au moins deux anticorps se liant à au moins deux épitopes différents sur lesdits oligomères de peptide amyloïde-β, le premier anticorps étant étiqueté avec un premier marqueur de fluorescence et l'au moins second anticorps étant étiqueté avec un second marqueur de fluorescence,
dans lequel ledit premier marqueur de fluorescence agit en tant que donneur transférant son énergie audit second marqueur de fluorescence agissant en tant qu'accepteur ;
d) déterminer l'intensité du signal de transfert d'énergie de résonance de fluorescence émis par ledit échantillon étiqueté par fluorescence pour détecter des oligomères de peptide amyloïde-β présents dans ledit échantillon corporel.

2. Procédé selon la revendication 1, dans lequel ledit fluide corporel est un fluide corporel de vertébré, de préférence provenant d'humains, de bétail, de chevaux, de chiens, de chats et de rongeurs.

3. Procédé selon la revendication 1 ou 2, dans lequel lesdits épitopes sont situés de préférence dans des positions d'acide aminé aa 1 - 24 à de l'extrémité amino-terminale, et dans lequel les épitopes sont de préférence sélectionnés dans le groupe constitué par un épitope en aa 4 - 13, un épitope en aa 17 - 24 ; un épitope en aa 1 - 5 ; un épitope en aa 1 - 11 ; un épitope en aa 1 - 7 ; un épitope en aa 15 - 24 ; un épitope en aa 3 - 9 ; un épitope en aa 11 - 26 ; un épitope en aa 4 - 10 ; un épitope en aa 13 - 28 ; un épitope en aa 1 - 10 ; un épitope en aa 31 - 40 ; un épitope en aa 8 - 17 ; un épitope en aa 15 - 30 ; un épitope en aa 17 - 24 ; un épitope en aa 1 - 28 ; un épitope en aa 12 - 28 ; un épitope en aa 17 - 42 ; un épitope en aa 20 - 40 ; un épitope en aa 37 - 42 ; un épitope en aa 8 - 17 ; un épitope en aa 11 - 28 ; un épitope en aa 1 - 6 ; un épitope en aa 8 - 17 ; un épitope en aa 12 - 28 ; un épitope en aa 25 - 35 ; un épitope en aa 15 - 30 ; un épitope en aa 17 - 26 ; un épitope en aa 1 - 12 ; un épitope en aa 32 - 40 ; un épitope en aa 33 - 42.

4. Procédé selon une ou plusieurs des revendications précédentes, dans lequel ledit démasquage est effectué en ajoutant un ou plusieurs détergents, de préférence des détergents anioniques, par exemple, SDS, du désoxycholate de Na ; des détergents non ioniques, par exemple, Triton X-100, Tween 20, Nonidet P-40 ; des détergents zwitterioniques, par exemple, CHAPS, et des détergents cationiques, par exemple, du bromure d'ammonium triméthyle tétradécyle (TTAB), et/ou d'autres agents de démasquage de dénaturation, de préférence des réactifs basiques ou acides, par exemple de l'acide formique, du chlorhydrate de guanidine ou de l'urée ou des hydroxydes d'alcali.

5. Procédé selon la revendication 4, dans lequel ledit détergent est utilisé en une concentration inférieure à la concentration de micelle critique, de préférence à environ 0,01 - 2 %, de préférence 0,02 - 1 % en poids.

6. Procédé selon une ou plusieurs des revendications précédentes, dans lequel la température de ladite étape de démasquage b) et/ou de ladite étape de mise en contact c) est dans la plage d'environ 15 °C - 40 °C, de préférence 17 °C - 30 °C, et dans lequel de préférence l'incubation avec lesdits agents de démasquage et/ou lesdits anticorps s'effectue pendant environ 30 - 90 minutes.

7. Procédé selon une ou plusieurs des revendications précédentes, dans lequel ledit fluide corporel est sélectionné dans le groupe constitué par le fluide cérébro-spinal, le sang, l'urine, les larmes et la salive.

8. Procédé selon une ou plusieurs des revendications précédentes, dans lequel ladite intensité dudit signal de transfert d'énergie de résonance de fluorescence est déterminée par une cytométrie de flux ou par des procédés photométriques.

9. Procédé selon la revendication 1, dans lequel les étapes (b) et (c) sont effectuées simultanément en un seul traitement.

10. Procédé selon une ou plusieurs des revendications précédentes, dans lequel on ajoute des inhibiteurs de protéase à la solution de démasquage et/ou à la solution contenant les anticorps.
